Europäisches Patentamt

**European Patent Office**

**Office européen des brevets**

(11) Publication number: **0 334 785**
**A2**

---

(12) **EUROPEAN PATENT APPLICATION**

(21) Application number: **89500003.2**

(22) Date of filing: **05.01.89**

(51) Int. Cl.⁴: **A 61 L 9/03**

(30) Priority: **04.03.88 ES 8800675**

(43) Date of publication of application:
**27.09.89 Bulletin 89/39**

(84) Designated Contracting States:
**AT BE CH DE FR GB GR IT LI LU NL SE**

(71) Applicant: **COOPER-ZELTIA, S.A.**
**Apartado 16**
**E-36400 Porrino Pontevedra (ES)**

(72) Inventor: **Rodriguez Besada, Maximino**
**Avenida de la Concordia, 8, 3**
**Tuy Pontevedra (ES)**

(74) Representative: **Gomez-Acebo y Pombo, José Miguel**
**c/o CLARKE, MODET & Co. Paseo de la Castellana 164**
**E-28046 Madrid (ES)**

(54) **Device for vaporizing thermically vaporizable substances.**

㊉ A device for vaporizing thermically vaporizable substances which consist of a housing (1) provided with electrical heating means designed to be fitted with the container (19) with the vaporizable substance and closed on the bottom by a cap (2) the cap (2) having an opening comprising a peripheral wall (16) directed towards the inside of which, at least part is provided inside with2 threading (18) to allow, in collaboration with the neck (20) with inner threading the fixing of the container (19) which holds the vaporizable substance thereby allowing the replacement of the container (19) to be made without the need to remove the cap (2).

FIG. 1

EP 0 334 785 A2

**Description**

## DEVICE FOR VAPORIZING THERMICALLY VAPORIZABLE SUBSTANCES

This invention relates to a device for vaporizing thermically vaporizable substances, and more specifically, for vaporizing thermically vaporizable substances having insecticide, deodorant, odorizing, bactericidal, etc., properties.

There are already known devices for this purpose, which consists of a housing provided with electrical heating means, in which a container holding the vaporizable substance is fitted. The housing consists of an upper cover and a lower cap which fasten together.

In the type of devices that have been mentioned, the container holding the thermically vaporizable substance is located inside the housing, making it necessary, each time the container needs to be replaced, to remove the cap, take out the empty container, put in the new container and place the lower cap in its original position.

The container for the vaporizable substance is provided with an absorbent body which projects out of the container which allows the vaporizable substance to be extracted by capillary action. The heating means are placed in such a way that the upper portion of the absorbent body is heated indirectly, for the purpose of causing controlled vaporization of the vaporizable substance.

In the type of devices referred to, the replacement of the container for the vaporizable substance requires, as has been mentioned, that the cap which closes the lower part of the housing be removed and then replaced to its original position. Also, these devices, to be connected to the mains are provided with a plug that is plugged in a shocket.

The object of this invention is to develop a device for the purpose mentioned, of simple design, in which the replacement of the container for the vaporizable substance can be made quickly and without the need to open or remove the lower cap of the housing.

Another object of the invention is to develop a device whose dimensions can be reduced considerably with respect to traditional devices.

A further object of the invention is to develop a device that can be plugged directly into an electrical outlet of a power supply, without having to use ancillary fixtures.

The device of the invention is characterized because the cap which closes the lower part of the housing is provided with an opening whose upper portion is surrounded by a wall. This wall has a cylindrical end section povided with a threaded inner surface. The mentioned opening and wall are dimensioned to receive the upper part of the container for the vaporizable substance. This container is provided with a threaded neck, which is screwed into the cylindrical section of the wall which surrounds the opening of the cap and which is provided with an inner threading.

With this desing, the container for the vaporizable substance is placed inside the housing and suspended from it by the threading of the neck of the container into the threaded section of the cylindrical wall which surrounds the opening of the lower cap of the housing.

According to another characteristic of the invention, the cover as well as the lower cap which make up the housing are provided with respective side notches facing one another which determine in one of the side walls of the housing an opening inside which an electrical plug is mounted which is held and tightly secured by the edges of said notches.

From the upper surface of the cap, which closes the bottom portion of the housing, two columns situated diametrically opposite to one another extend upwards, outside the threaded section of the wall which surrounds the opening of the caps which serve as supports for the connection of the electrical heating elements, which will consist of an electrical resistance, whose on or off condition is controlled by a switch which is mounted on the side of the housing. The device can also include an indicator light which will be mounted on a visible area of the housing and will serve to indicate whether the device is on or off.

In order to be able to better understand the characteristics and advantages of the device of this invention, a more detailed description will be given of it as follows, with reference to the drawings attached, in which one possible embodiment is represented, given as a non limitative example.

In the drawings:

Figure 1 is a perspective exploded view of a device constructed according to the invention.

Figure 2 is a section of the cover of the housing, through the line A-A of Figure 1.

The device of the invention comprises a housing which is made of an upper cover 1, opened at its lower base, and a closing cap 2 which fits into and is fastened under the cover 1 to close up the cover 1.

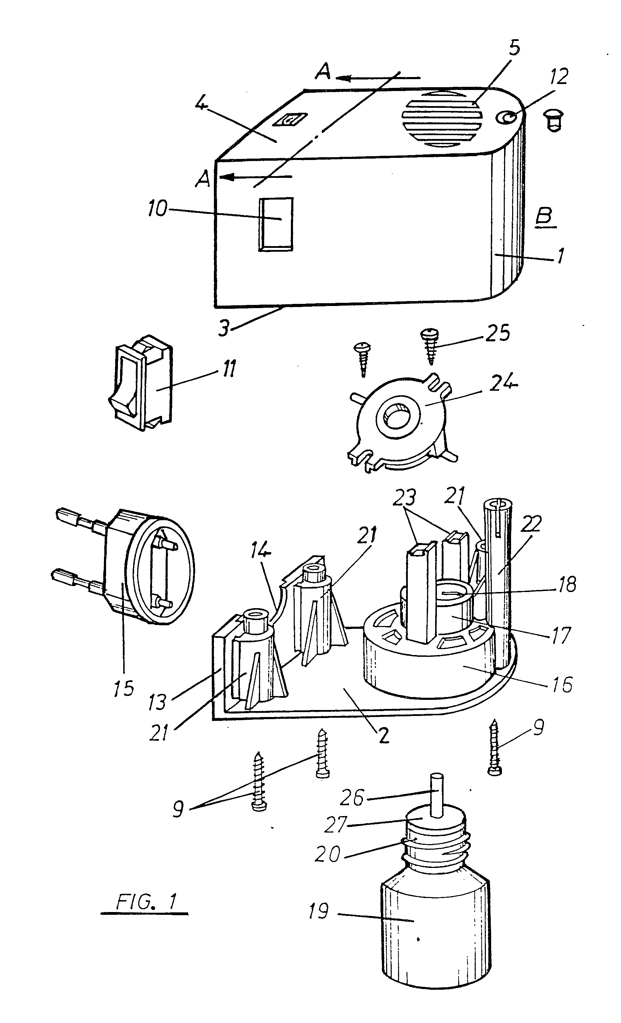

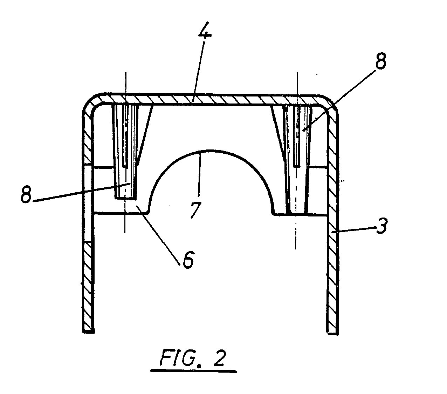

As can be seen in Figure 1 and 2, cover 1 includes a side wall 3 and an upper wall or bottom 4. The upper wall or bottom 4 is provided with a vent grid 5 for the escape of the vapors of the vaporizable substance. The flat transverse wall 6, Figure 2, is not as high as the rest of side wall 3 and is provided with a more or less arched semicircular notch in its center. From the upper wall or bottom 4 two pins 8 extend outward to receive the attachment screws 9 from the lower cap 2. Side wall 3 of cover 1 is provided with a window 10 inside which the switch 11 is mounted. Also in the upper wall of bottom 4 there is hole 12, through which an indicator light is visible.

The lower cap 2, which has the same contour as the cover 12, has in its straight cross edge an upper wall provided with an arched edge notch 14 which faces the notch 7 of the cover 1. When the cap 2 is fitted under cover 1, the notches 7 and 14 which face one another form an opening through which the plug 15 is mounted, with the plug tightly held in place by the edges of the notches 7 and 14.

The lower cap 2 is also provided with an opening whose top part is surrounded by a wall which includes the first section 16, adjacent to the cap 2, and a second cylindrical section 17 provided with inner threading 18. The opening and section of the wall 16 determine a contour approximately the same as that of the container 19 for the vaporizable substance. This container is provided with a threaded neck 20, with teh second section 17 of the wall having the same diameter and threading as the neck 20.

The lower cap 2 is provided with two tubular columns 21 on its interior surface through which the attachement screws 9 for securing said cap to columns 8 of the cover 1 are introduced. Also, a column 22 provided on its upper portion with a hollow section to receive the indicator light 12, extends up from the interior surface of cap 2. Lastly, from the step defined between the sections 16 and 17 of the wall which surrounds the upper portion of the opening of the casing 2, two columns 23 extend upward, on which a support 24 is mounted, with screws 25, the support 24 being the support of the electrical resistance which defines the heating means for the vaporizable substance.

In the container 19 of the vaporizable substance, once its cap is removed, there is an absorbent means which may adopt for example the form of a wick 26 which penetrates inside the container in order to allow the vaporizable substance to escape by means of capillary action. The wick 26 is provided with a disk 27 which closes the top edge of the neck 20.

The cap 2 is held underneath the cover 1 by the screws 9 which pass through the tubular columns 21 and screw into the pins 8 of the cover 1. By inserting the cap 2 into the cover 1 the plug 15 is held in place. Previously, the support 24 which holds the heating means is mounted on columns 23 and the indicator light 12 is mounted on column 22.

To operate the device of the invention, once the container 19 of the vaporizable substance is opened and the wick 26 is in place, the container 19 fits into the opening of the cap 2 from underneath, and its neck 20 is screwed into the inner threads 18 of the cylindrical section 17 of the said wall thereby holding and securing in place the container 19 inside the device of the invention: The wick 26 passes through the center hole of the support 24. With this configuration, as soon as the device is connected to an electrical supply and the switch 11 is flipped on, activating the heating means 24, the vaporization of the substance begins, with the vapor escaping through the vent grid 5.

The plug 15 being solidary with the housing, the device can be secured directly into a socket of a power source.

To replace the container 19 all that has to be done is unscrew it from the device and screw in a new container filled with the vaporizable substance.

Having been sufficiently disclosed the nature of the invention, as well as how it is embodied, it should be mentioned that the lay-out that has been indicated, and illustrated in the attached drawings, may be subject to detail modifications provided that the basic principle is not altered.

## Claims

1. A device to thermically vaporize thermically vaporizable substances, which consists of a housing provided with electrical heating means, on which a container of the vaporizable substance fits, with the housing consisting of an upper cover and a lower closing cap which attaches to said cover, characterized because the said lower cap is provided with an opening that is surrounded in its upper portion by a wall, which shows a cylindrical section threaded on its interior surface, with said opening and wall dimensioned to receive from underneath the upper part of the container of the vaporizable substance, whose threaded neck screws inside the threaded section of said wall, from which the container is suspended, with the electrical heating means situated above the edge of the wall which surrounds said opening.

2. A device according to claim 1, characterized because the cover and lower cap are made with their respective side notches facing one another which in one of the side walls determine an opening in which an electrical plug is mounted, with the body of said plug held in place by the edges of the notches.

3. A device according to claim 1, characterized because the wall which surrounds the opening in said lower cap is provided with a first section adjacent to the cap, which has a contour which is approximately the same as that of the container of the vaporizable substance, and a second cylindrical section with internal threads, whose threading end section is the same as the neck of said container; with two columns extending up on the outside of said wall positioned diametrically oposite one another on each side of the cylindrical section, which serve as supports for the fixing the electrical heating means.

FIG. 1

FIG. 2